# EUROPEAN PATENT APPLICATION

(11) **EP 1 415 606 A2**
(43) Date of publication of application: **06.05.2004**
(21) Application number: 03256880.0
(22) Date of filing: 30.10.2003
(51) Int. Cl.: A61B 17/80, A61B 17/70

(54) **Universal washer assemblies for polyaxial bone stabilizers**

(30) Priority: 31.10.2002 US 284606
(71) Applicant: DePuy AcroMed, Inc., Raynham, MA 02767-0350 (US)
(72) Inventor: Borgstrom, Amie, North Attleborough MA 02760 (US); Dunbar, William, Jr, Norton MA 02766 (US)
(74) Representative: Alton, Andrew

(57) **Abstract**

Devices for spinal fixation are disclosed, and especially those used for joining a bone anchor with spinal stabilizers such as a spinal rod or spinal plate (50) in a polyaxial fashion. More specifically, polyaxial washer assemblies are described which incorporate arcuate washers (10,20) and which may be used to retrofit spinal stabilizers that may not have been designed for polyaxial application since the washer assemblies may be used across a number of differing aperture sizes.

## Description

This invention relates to devices for bone fixation, in particular, devices for joining a bone anchor with bone stabilizers such as a spinal rod or bone plate in a polyaxial fashion through use of a universal polyaxial washer assembly.

It is well know and practiced that when securing and maintaining bones in a preferred alignment, a plurality of bone anchors may be attached to one or more bones and subsequently secured together in a desired orientation with a bone stabilizer. Examples of bone stabilizers include "longitudinal members," and in some cases, "connectors" as is known in the art. That is, the longitudinal member (e.g. a rod or a plate) connects the bone anchors together, and (if needed), the connectors are used to secure the bone anchor to the longitudinal member. Accordingly, there are at least two criteria that would be desirable to be satisfied when securing bones in a desired alignment using bone anchors and a bone stabilizer:
(I) it is desirable to attach each bone anchor to a bone in a manner that will inhibit movement of the anchor relative to the bone. Thus, the orientation of the bone anchor to its attached bone may be dependent on, e.g., the bone configuration, density, and/or fractures therein;
(ii) the bone stabilizer must be attached to each bone anchor, and in particular, to an included shaft, in a manner so that the longitudinal member (of the bone stabilizer) is oriented to effectively maintain the desired alignment of bones.

In order to satisfy the above criteria, it may be preferable that the shafts of the bone anchors are not parallel to each other and/or not perpendicular to the longitudinal member when they are attached to their respective bones. Moreover, for each bone alignment procedure performed using bone anchors and bone stabilizers, the orientations of the shafts relative to one another and the longitudinal member may be substantially unique.

Accordingly, a preferable position of the longitudinal member can be compromised by the various orientations of the bone anchoring shafts, these various orientations due to, e.g., the preferred positions of the anchors when they are secured to the bones. Thus, it is advantageous to have the ability to firmly secure such bone anchoring shafts to a bone position retainer, wherein the shafts may be at various orientations to the longitudinal member, i.e., in polyaxial or multi-angular orientations.

U.S. Pat. No. 5,984,924 discloses such a bone alignment system capable of multi-angular orientations with respect to the bone anchor and longitudinal member. Through use of a system of concave or convex arcuate washers, the multi-angular orientations are achieved.

U.S. Pat. No. 6,315,779 discloses a multi-axial bone fixation implant having an elongated member, one or more bone anchor assemblies, and stabilizer members which are fitted within the elongated member. The stabilizer members are rectilinear in shape and do not include arcuate surfaces. The only use of washers disclosed are ones having an undercut within its oblong aperture on the top of the implant. The implant is locked by a nut and the washer atop the elongated member, the enlarged portion of a bone anchor is forced against an inside wall of the stabilizer, which is in turn locks against the elongated member. Disadvantages of this system are the large number of separate parts and that the washers can be cumbersome to use in that they are not secured to the elongated member.

The present invention provides an advance in the art by providing a universal polyaxial washer assembly which can secure itself through an aperture, the aperture not requiring any special channel to secure the universal washer assembly. Once the universal washer assembly is secured through the aperture of a bone plate, or through the aperture of a bone anchor to bone stabilizer connector, the number of parts a surgeon handles during a surgery in minimized while gaining the advantage of multiple orientations provided by use of the arcuate washers.

According to a first aspect of the invention, there is provided a polyaxial washer assembly comprising: a first washer comprising a convex surface, an opposite non-convex surface and a throughbore; a second washer comprising a convex surface, an opposite non-convex surface and a throughbore; and a connecting tube having a first end and a second end, the first end for inserting into the first washer throughbore and the second end for inserting into the second washer throughbore whereby the first and second ends engage their respective washers.

A preferred embodiment of the invention includes the assembly described above further comprising a body for connecting a bone anchor to a bone stabilizer or for connecting as least two bone anchors (such as a bone plate) wherein the body comprises a top surface and a bottom surface and an aperture passing through the body from the top surface to the bottom surface wherein the first washer is positioned on the top surface and the second washer is position on the bottom surface and the connecting tube connecting the first and second washers passes through the aperture.

A further aspect of the invention provides an implantable medical washer assembly kit, comprising: a plurality of washer assembly components comprising washers, straight tubes and angled tubes, the washers configured to receive and engage the straight or angled tubes and the components being of various sizes.

A further aspect of the invention provides a system for spinal stabilization comprising a spinal rod or bone plate; a bone anchor comprising a bone engaging first portion, a machine threaded second portion for engaging a locking nut, and a third portion, intermediate to the first and second portions, comprising arcuate surfaces for polyaxial engagement with a connector; and a locking nut, wherein, the bone anchor is joined with the rod or bone plate by connecting bodies incorporating the washer assembly aspect of the invention.

The invention will be more fully understood from the following detailed description taken in conjunction with the accompanying drawings, in which:
Fig. 1 depicts a universal polyaxial washer assembly of this invention for an angled tube embodiment;
Fig. 2 depicts a straight tube component embodiment of the invention;
Fig. 3 depicts an angled tube component embodiment of this invention; and
Fig. 4 depicts a straight tube embodiment of this invention wherein one end of the straight tube is integral to one of the washers.

Generally, this invention relates to assemblies for spinal fixation especially for those used for joining a bone anchor with spinal stabilizers such as a spinal rod or spinal plate in a polyaxial fashion. More specifically, the invention is directed to polyaxial washer assemblies which incorporate arcuate washers and which may be used to retrofit spinal stabilizers that may not have been designed for polyaxial application since the washer assemblies of this invention may be used across a number of differing aperture sizes not requiring any special modification for receipt of the washer assemblies of this invention.

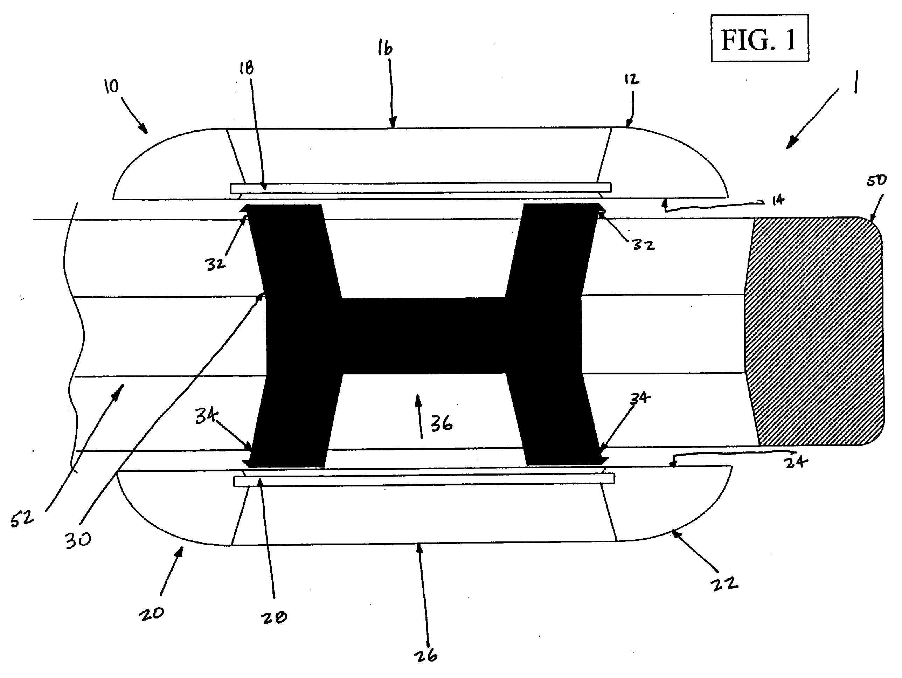

Fig. 1 depicts a universal polyaxial washer assembly of this invention for an angled tube embodiment. Polyaxial washer assembly 1 comprises first washer 10 comprising a convex surface 12, an opposite non-convex surface 14 and a throughbore 16; a second washer 20 comprising a convex surface 22, an opposite non-convex surface 24 and a throughbore 26; and a connecting tube 30 having a first end 32, a second end 34, and throughbore 36. Connecting tube end 32 is inserted into and engages first washer throughbore 16 and connecting tube end 34 is inserted into and engages second washer throughbore 26. Assembly 1 is assembled on either sides of aperture 52 of plate or connector 50. In this embodiment tube ends 32 and 34 are designed to be engaged into receipts 18 and 28 of washers 10 and 20, respectively.

While Fig. 1 and the remaining figures may represent the washer component of this invention as concave, it should be apparent to one skilled in the art that this invention contemplates other arcuate washer surfaces being be used according to this invention such as convex surfaces. Such surfaces will engage with body anchors as disclosed and depicted, for example, in U.S. Pat. No. 5,984,924 or in U.S. Pat. No. 6,315,779 the disclosures of which are incorporated in their entireties.

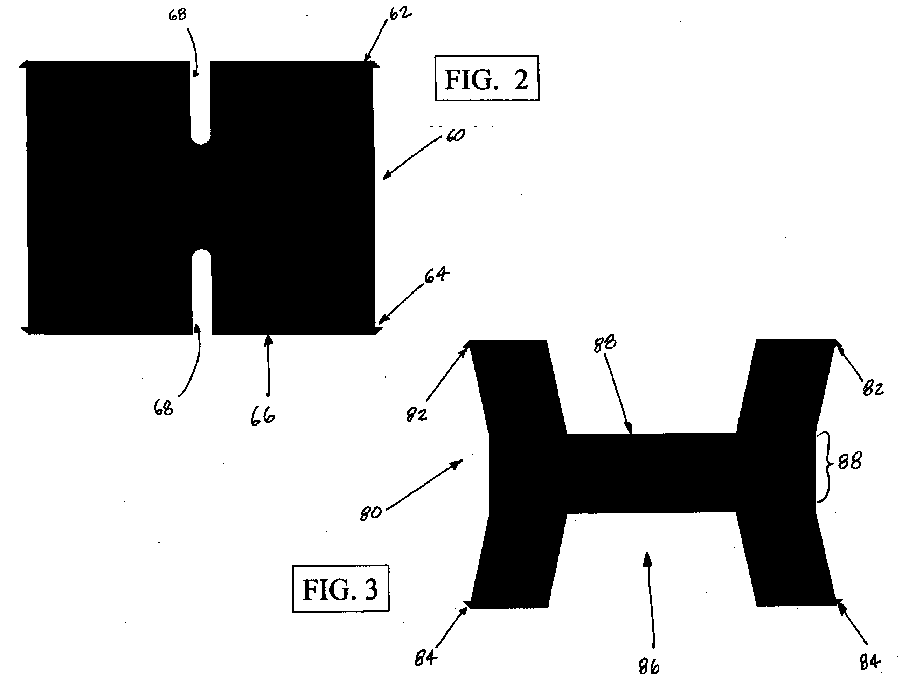

Fig. 2 depicts straight tube 60 component of this invention. Tube 60 comprises first end 62, second end 62, and throughbore 66. Ends 62 and 64 are designed to be inserted into and engaged into receipts 18 and 28 of washers 10 and 20 (not shown). Ends 62 and 64 are made flexible or resilient due to slots 68. The cross sectional width of throughbore 66 should be sufficient to allow angulation of the bone anchor.

Fig. 3 depicts a detailed drawing of the angled tube 80 component of this invention. Tube 80 comprises first end 82, second end 84, and throughbore 86. Ends 82 and 84 are designed to be engaged into receipts 18 and 28 of washers 10 and 20 (not shown). Tube 80 contains central portion 88 located between end 82 and 84. Central portion 88 is of narrower diameter than ends 82 and 84 so that angulation of a bone anchor may be achieved over a wider range of motion. The diameter of central portion 88 is desirably sized to fit within the aperture of the connecting device that the washer assembly is used with. Angled tube 80 differs from straight tube 60 in that slots are not needed to assist in flexibility or resiliency of tube 80 since ends 82 and 84 are separated by a relatively large open area.

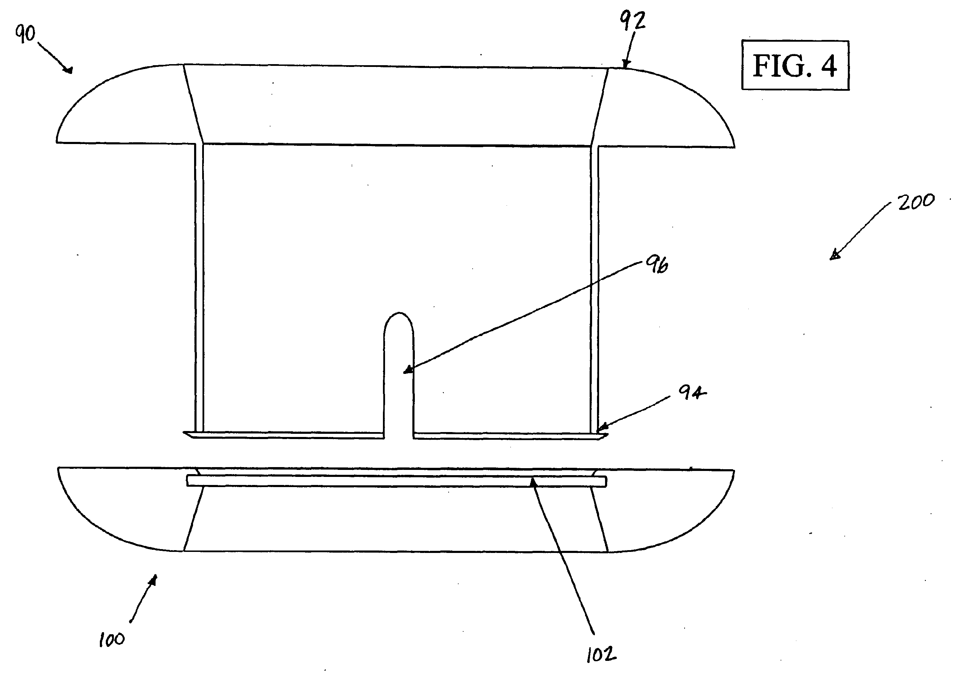

Fig. 4 depicts yet another embodiment of this invention. Referring to Fig. 4, washer assembly 200 comprises integral washer and tube 90 having washer component end 92 and open tube end 94. Slots 96 afford flexibility for insertion and engagement of end 94 into receipt 102 of washer 100.

For all embodiments, the washer assemblies may be assembled as part of the manufacturing process (i.e., components shipped assembled) or assembled in the operating room just prior to implantation. Assembly in the operating room is desirable if the washer assemblies are being used to "retrofit" connectors or plates that originally were not designed for use with polyaxial washer assemblies such as though described and claimed in this invention. Thus, in this sense, the washer assemblies of this invention may be used with a multitude of connectors and are therefore referred to as "universal".

The components of this invention's assemblies may be made of any medical grade metal (e.g., titanium or stainless steel). Additionally, the regarding the tube component itself, an alternate material such as carbon fiber or a memory metal may be used to facilitate ease of assembly.

Another embodiment of this invention relates to the assemblies described above further comprising a body for connecting a bone anchor to a bone stabilizer (e.g., a rod) or for connecting as least two bone anchors (e.g., a bone plate) wherein the body comprises a top surface and a bottom surface and an aperture passing through the body from the top surface to the bottom surface wherein the first washer is positioned on the top surface and the second washer is position on the bottom surface and the connecting tube connecting the first and second washers passes through the aperture.

In use, the washer assemblies of this invention are contemplated for use with polyaxial bone anchors that are well known in the art. Such bone anchors generally comprise a bone engaging threaded first portion, a machine threaded second portion for engaging a locking nut, and a third portion, intermediate to the first and second portions, comprising arcuate surfaces (for example, as depicted in Fig. 4 of U.S. Pat. No. 5,984,924 or in Fig. 7 of U.S. Pat. No. 6,315,779, the disclosures of which are incorporated by reference in their entirety) for polyaxial engagement with connectors or bone plates incorporating the washer assemblies of this invention. Thus, the invention also relates to systems for spinal stabilization comprising a spinal rod; a bone anchor comprising a bone engaging first portion, a machine threaded second portion for engaging a locking nut, and a third portion, intermediate to the first and second portions, comprising arcuate surfaces for polyaxial engagement with a connector; and a locking nut used in combination with the connecting devices of this invention as described above. Additional systems of this invention include a system for spinal stabilization comprising at least two bone anchors comprising a bone engaging first portion, a machine threaded second portion for engaging a locking nut, and a third portion, intermediate to the first and second portions, comprising arcuate surfaces for polyaxial engagement with a connector; and a locking nut, wherein, the bone anchors are joined by the connecting device comprising a plate having an aperture for receiving and retaining a washer.

Yet, another aspect of this invention relates to an implantable medical washer assembly kit, comprising: a plurality of washer assembly components comprising washers, straight tubes and angled tubes, the washers configured to received and engage the straight or angled tubes. Desirably, the kit is comprised of washers, straight tubes and angled tubes, the washers configured to received and engage the straight or angled tubes of various sizes. Additionally the kits may further comprise spinal rod and bone anchor connectors and bone plates.

It should be understood that the foregoing disclosure and description of the present invention are illustrative and explanatory thereof and various changes in the size, shape and materials as well as in the description of the preferred embodiment may be made without departing from the invention.

## Claims

1. A polyaxial washer assembly comprising:
a first washer comprising a convex surface, an opposite non-convex surface and a throughbore;
a second washer comprising a convex surface, an opposite non-convex surface and a throughbore; and
a connecting tube having a first end and a second end, the first end for inserting into the first washer throughbore and the second end for inserting into the second washer throughbore whereby the first and second ends engage their respective washers.

2. The assembly of claim 1, wherein the tube is a straight tube having first and second ends.

3. The assembly of claim 1, wherein the tube comprises a first end having an angled section, a center straight section, and a second end having an angled section.

4. The assembly of claims 2 or 3, wherein the first and second tube ends have slits to allow for deflection or deformation of the tube ends for engagement into their respective washers.

5. The assembly of any preceding claim, further comprising a body for connecting a bone anchor to a bone stabilizer or for connecting at least two bone anchors wherein the body comprises a top surface and a bottom surface and an aperture passing through the body from the top surface to the bottom surface wherein the first washer is positioned on the top surface and the second washer is position on the bottom surface and the connecting tube connecting the first and second washers passes through the aperture.

6. The assembly of claim 1, wherein the first end of the tube is integral with the non-convex surface of the first washer.

7. The assembly of claim 1, wherein at least one of the washers has a convex surface instead of a concave surface.

8. An implantable medical washer assembly kit, comprising:
a plurality of washer assembly components comprising washers, straight tubes and angled tubes, the washers configured to received and engage the straight or angled tubes.

9. The kit of claim 8 wherein, the washer assembly components are comprised of washers, straight tubes and angled tubes, the washers configured to received and engage the straight or angled tubes of various sizes.

10. The kit of claims 8 or 9 further comprising apertured connectors and bone plates.

11. A system for spinal stabilization comprising:
a spinal rod;
a bone anchor comprising a bone engaging first portion, a machine threaded second portion for engaging a locking nut, and a third portion, intermediate to the first and second portions, comprising arcuate surfaces for polyaxial engagement with a connector; and
a locking nut, wherein, the bone anchor and rod are joined by the connecting bodies of claim 5.

12. A system for spinal stabilization comprising at least two bone anchors comprising a bone engaging first portion, a machine threaded second portion for engaging a locking nut, and a third portion, intermediate to the first and second portions, comprising arcuate surfaces for polyaxial engagement with a connector; and a locking nut, wherein, the bone anchors are joined by the bone plate of claim 5.
